# EUROPEAN PATENT SPECIFICATION

(11) **EP 0 991 409 B1**
(45) Date of publication and mention of the grant of the patent: **30.01.2002**
(21) Application number: 98937738.7
(22) Date of filing: 30.07.1998
(51) Int. Cl.: A61K 31/445, A61K 9/20

(54) **CONTROLLED RELEASE PHARMACEUTICAL COMPOSITIONS CONTAINING TIAGABINE**
TIAGABINHALTIGE ARZNEIZUBEREITUNGEN MIT GESTEUERTER WIRKSTOFFVERABREICHUNG
COMPOSITIONS PHARMACEUTIQUES A LIBERATION LENTE, CONTENANT DE LA TIAGABINE

(30) Priority: 01.08.1997 US 54432 P; 08.08.1997 IE 970588
(43) Date of publication of application: 12.04.2000
(73) Proprietor: ELAN CORPORATION, Plc, Dublin 2 (IE)
(72) Inventor: CLANCY, Maurice, Joseph, Anthony, Athlone, County Westmeath (IE); CAULFIELD, Michelle, Ballyhaunis, County Mayo (IE); CUMMING, Kenneth, Iain, Phibsboro, Dublin 7 (IE)
(74) Representative: Ryan, Anne Mary
(86) International application number: IE9800067
(87) International publication number: WO9906045

(56) References cited:
- WO-A-87/00171
- WO-A-95/29665
- WO-A-95/31976
- WO-A-96/34606
- WO-A-97/02813
- WO-A-97/43902
- WO-A-97/47619
- WO-A-98/05330

## Description

### Technical Field

This invention relates to controlled release tiagabine formulations and in particular to matrix delivery systems for providing oral controlled release tiagabine formulations, including once or twice daily formulations.

### Background Art

The anti-epileptic drug tiagabine hydrochloride, a nipecotic acid derivative linked to a lipophilic anchor which enables it to cross the blood-brain barrier, potently and specifically inhibits uptake of the inhibitory neurotransmitter γ-aminobutyric acid (GABA) into astrocytes and neurons. Tiagabine is primarily under investigation as an anticonvulsant agent, but the compound is also reported to possess anxiolytic activity and may be beneficial in the treatment and prevention of tardive dyskinesia. Tiagabine has shown broad activity against a range of seizure types, including drug-induced, electroshock-induced, light-induced, amygdala-kindled, and audiogenic and has been approved in a number of countries for add-on treatment of adult epileptic patients with complex partial seizures. It is well tolerated and does not cause withdrawal effects or displace other drugs. In humans, tiagabine absorption is rapid and complete. It is metabolized in the liver with a linear process of elimination and a half-life of 5-8 hours. Although tiagabine does not induce or inhibit metabolic processes, it can provide a target for enzyme inducers that can lower its elimination half-life to 2-3 hours. Conventional formulations of tiagabine, such as 10mg formulations, are dosed t.i.d. or q.i.d.

Published information regarding therapeutic plasma levels for tiagabine include Leach et al. (*Seizure* 4(2):155-7, 1995), which reports an experience with a deliberate overdose of tiagabine. Plasma levels were 3.1 mg/ml four hours after ingestion which is 30 times higher than typical steady state therapeutic dosing levels. Sachdeo et al. *(Archives of Neurology* 54(5):595-601, 1997) evaluated 2 regimens for tiagabine dosing, 16mg b.i.d. or 8mg q.i.d. (34 mg/day), as add-on therapy for patients with complex partial seizures. Schacter (*Epilepsia* 36 Suppl 6:S2-26, 1995) assessed tiagabine monotherapy in patients with partial seizures. In a dose-ranging study, the mean final dose was 38.4 mg/day (range: 24-54 mg/day) for patients who converted to tiagabine monotherapy. In a low (6 mg/day) versus high (36 mg/day) dose study, median 4-week complex partial seizure rates decreased significantly in patients from both dose groups but significantly more patients in the high-dose group experienced a reduction in seizures of at least 50% compared with the low-dose group.

US Patent No. 5,010,090 discloses a class of novel compounds, including tiagabine, and their pharmaceutically acceptable salts that exhibit GABA-uptake inhibitory properties useful for the treatment of epilepsy and other CNS related diseases. US Patent No. 5,354,760 discloses crystalline tiagabine hydrochloride monohydrate and a process for preparing the same. WO 96/34606 discloses tiagabine hydrochloride compositions which include an antioxidant such as α-tocopherol to stabilize the tiagabine. Despite the relatively short elimination half-life for tiagabine in humans and the concomitant need to administer the drug several times per day, none of these patents address the additional benefits possible from a controlled-release formulation for tiagabine such as enhanced patient compliance or improved therapeutic results.

WO 95/29665 discloses a slow-release extended antiepileptic drug dosage form that has an exit in the dosage form for releasing the antiepileptic drug and a lamina between the dosage form wall and the antiepileptic drug formulation to maintain the integrity of the dosage form during the delivery of the antiepileptic drug to the patient. Additionally, this application discloses a diffusion-dosage form that releases a drug by membrane-controlled diffusion, a bioerodible dosage form and a ion-exchange dosage form for administration of antiepileptic drugs selected from the group consisting of hydantoins, barbiturates, deoxybarbiturates, iminostilbenes, succinimides, oxazolidinediones and benzodiazepines. Examples for the antiepileptic drugs phenytoin, carbamazepine, and ethotoin are provided.

Controlled-release therapeutic dosage forms for tiagabine in which the medicinal substance is incorporated into a matrix would be desirable *per se* on account of the ease of their manufacture, the low degree of variation between different manufacturing processes and because of the relatively low costs. Ideally, not only should the dosage form control release of the tiagabine in such a manner that an effective concentration in the blood can be maintained over an extended period of time, but also the drug release should be such that the drug concentration in the blood remains relatively constant over the extended period of time to improve therapeutic results and/or minimize side effects. Thus, there exists a need for a controlled-release tiagabine formulation having these properties, especially one having minimal Cₘₐₓ to Cₘᵢₙ peak to trough variations over a period of at least either 12 or 24 hours.

It is an object of the present invention to provide controlled release oral dosage forms for tiagabine which provide therapeutic levels of tiagabine for a period of at least 12 hours, preferably 24 hours or longer.

### Disclosure of Invention

The above-mentioned object and others are achieved by virtue of the present invention, which provides a controlled release oral pharmaceutical preparation comprising a therapeutically effective amount of tiagabine or a pharmaceutically acceptable salt thereof dispersed in a rate controlling polymeric matrix comprising at least one rate controlling polymer, which preparation provides therapeutically effective plasma levels of tiagabine for a period of at least 12 hours, and which provides a mean *in vitro* dissolution profile in aqueous media such that about 5 to 40% of the tiagabine is released after 1 hour; about 25 to 65% of the tiagabine is released after 4 hours; about 55 to 95% of the tiagabine is released after 10 hours and about 80 to 100% of the tiagabine is released after 22 hours when carried out in a USP Apparatus (II) (paddles) at 50 r.p.m. in deionised water at 37°C as dissolution medium.

It is found that incorporation of tiagabine in the polymeric matrixes according to this invention allows for effective control over the release of tiagabine over time such that administration of the preparation achieves therapeutic levels of tiagabine over extended periods of time in humans, such as for at least 12 hours, and in certain preferred embodiments, for 24 hours or longer.

The applicants have found in the case of the formulations of the present invention that therapeutically effective blood levels of tiagabine can be maintained substantially over 24 hours with peak plasma levels occurring between 2 and 18 hours, preferably between 4 and 16 hours, most preferably between 6 and 14 hours.

As used herein, the term "tiagabine" refers to N-(4,4-di(3-methylthien-2-yl)but-3-enyl) nipecotic acid, including the R and S isomers and racemic mixtures, or a pharmaceutically acceptable salt thereof. The preferred pharmaceutically acceptable salt is the hydrochloride salt. The R isomer of N-(4,4-di(3-methylthien-2-yl)but-3-enyl) nipecotic acid is preferred and the monohydrate crystalline form of the R isomer of N-(4,4-di(3-methylthien-2-yl)but-3-enyl) nipecotic acid hydrochloride is the most preferred form of tiagabine.

By "controlled release" it is meant for purposes of the present invention that therapeutically active tiagabine is released from the preparation at a controlled rate such that therapeutically beneficial blood levels (but below toxic levels) of tiagabine are maintained over an extended period of time, e.g., providing a 12 hour or a 24 hour dosage form.

Preferably, the preparation releases tiagabine *in vivo* such that the duration over which the tiagabine plasma concentration is equal to or greater than 50% of the peak plasma concentration (maximum plasma concentration (Cmax)) is 10 hours or greater, more particularly at least 15 hours and especially at least 20 hours.

Further, preferably, the *in vivo* maximum plasma concentration minus the minimum plasma concentration divided by the average plasma concentration [(Cmax-Cmin)/Cav] taken over the effective period (at least 12 hours, preferably 24 hours) is less than 0.80, more preferably less than 0.60.

The invention provides controlled release oral dosage forms for tiagabine which provide therapeutic levels of tiagabine for a period of at least 12 hours, preferably 24 hours, having a mean *in vitro* dissolution profile in aqueous media at 37°C such that about 5 to 40% of the tiagabine is released after 1 hour; about 25-65% is released after 4 hours; about 55-95% is released after 10 hours and about 80-100% is released after 22 hours when measured according to the USP Apparatus II (paddles) method. More preferably, the *in vitro* dissolution profile shows release of about 10 to 30% tiagabine after 1 hour; 30 to 60% release after 4 hours; 60 to 90% release after 10 hours and 85 to 100% release after 22 hours.

Preferably, the at least one rate controlling polymer is selected from hydroxypropylmethylcellulose, hydroxyalkylcellulose, alkylcellulose, poly(ethylene)oxide, carboxymethylcellulose, hydrophilic cellulose derivatives, polyethylene glycols, polyvinylpyrrolidone, or mixtures thereof.

Further, preferably, the rate controlling polymer includes a hydroxypropyl-methylcellulose (HPMC), a hydroxypropylcellulose (HPC), a poly(ethylene oxide), an ethylcellulose or a combination thereof present in an amount of 5 to 75% by weight, more preferably 20 to 50% by weight, most preferably 30 to 45% by weight in the preparation.

In an especially preferred embodiment, the at least one rate controlling polymer comprises from 19 to 31% by weight of a hydroxypropylmethylcellulose and from 9 to 15% by weight of a hydroxypropylcellulose.

An especially preferred type of HPMC for use in accordance with the invention is an HPMC sold under the trademark Methocel (Dow Chemical Co.) or equivalents. Suitable Methocels include the K grades such as Methocel K15M, Methocel K100M, Methocel K100LV and Methocel K4M. Other suitable Methocels include the E, F and J grades. An especially preferred type of HPC for use in accordance with the invention is an HPC sold under the trademark Klucel (Hercules, Inc.) or equivalents. Suitable Klucels include Klucel LF, Klucel JF, Klucel GF, Klucel MF and Klucel HF. An especially preferred type of poly(ethylene oxide) for use in accordance with the invention is a poly(ethylene oxide) sold under the trademark Sentry Polyox (Union Carbide Corp.) or equivalents. Suitable Polyoxs include the Polyox WSR grades such as Polyox WSR Coagulant, Polyox WSR-301, Polyox WSR-303, Polyox WSR N-12K, Polyox WSR N-60K, Polyox WSR-1105, Polyox WSR-205 and Polyox WSR N-3000. An especially preferred type of ethylcellulose for use in accordance with the invention is an ethylcellulose sold under the trademark Ethocel (Dow Chemical Co.) or equivalents.

### Brief Description of Drawings

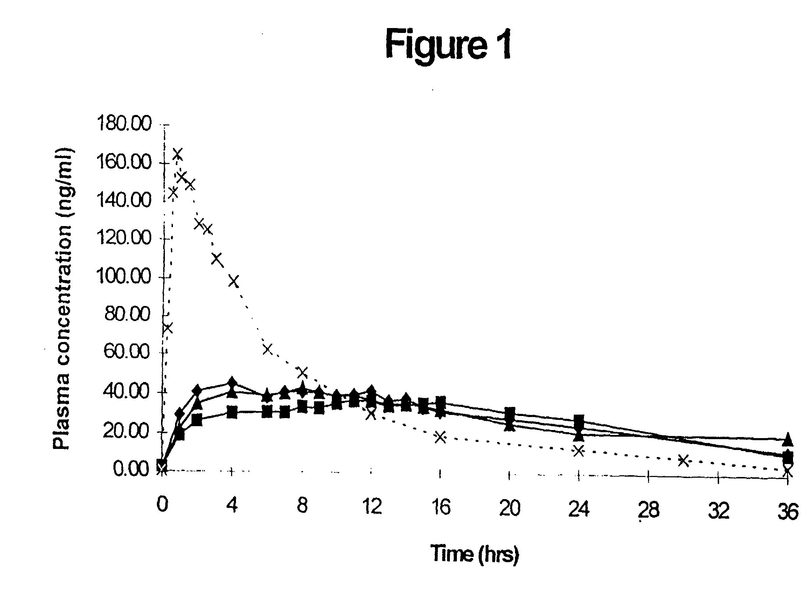

Figure 1 shows the tiagabine plasma level concentration (ng/ml) over 36 hours following administration of the following 10 mg tiagabine formulations made according to this invention to 12 healthy male volunteers in an open label, randomized, four period cross-over study: Formulation A (―◆―) [Batch 8 of Example 4; 20%wt Methocel K15M, 10%wt Klucel LF, Direct Compression]: Formulation B (―■―) [Batch 21 of Example 9, 30%wt Methocel K15M, 15%wt Klucel LF, Melt Granulation Type B]; and Formulation C (―Δ―) [Batch 22 of Example 9, 20%wt Methocel K15M, 10%wt Klucel LF, Melt Granulation Type B]. The control for this study is the commercially available immediate release tiagabine 10 mg Gabitril (Gabitril is a trade mark) tablets (Novo Nordisk) (―X―); and

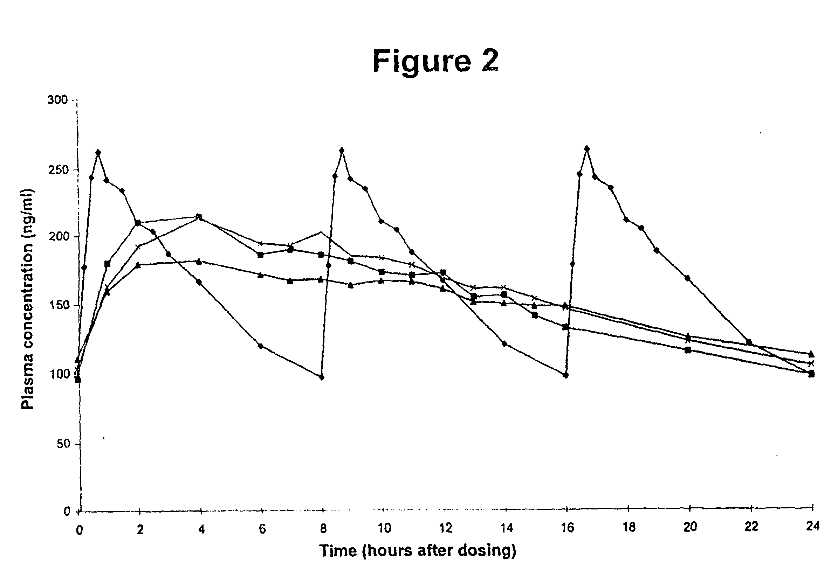

Figure 2 illustrates projected steady state plasma tiagabine profiles for Formulation A (―■―) [Batch 8 of Example 4; 20%wt Methocel K15M, 10%wt Klucel LF, Direct Compression]: Formulation B (―Δ―) [Batch 21 of Example 9, 30%wt Methocel K15M, 15%wt Klucel LF, Melt Granulation Type B]; and Formulation C (―X―) [Batch 22 of Example 9, 20%wt Methocel K15M, 10%wt Klucel LF, Melt Granulation Type B] at 30mg dosing once daily compared to the conventional Formulation D (―◆―) [immediate release tiagabine 10 mg Gabitril (Gabitril is a trade mark) tablets (Novo Nordisk)] dosed 10mg t.i.d.

The term "rate controlling polymer" as used herein includes hydrophilic polymers, hydrophobic polymers or mixtures of hydrophilic and/or hydrophobic polymers that are capable of retarding the release of tiagabine *in vivo* when tiagabine is dispersed in a polymeric matrix formed from the rate controlling polymers. Examples of rate controlling polymers to be used in this invention include hydroxyalkylcellulose, such as hydroxypropylcellulose and hydroxypropylmethylcellulose; poly(ethylene)oxide; alkylcellulose such as ethylcellulose and methylcellulose; carboxymethylcellulose; hydrophilic cellulose derivatives; polyethylene glycol; polyvinylpyrrolidone; cellulose acetate; cellulose acetate butyrate; cellulose acetate phthalate; cellulose acetate trimellitate; polyvinylacetate phthalate; hydroxypropylmethylcellulose phthalate; hydroxypropylmethylcellulose acetate succinate; poly(alkyl methacrylate); and poly (vinyl acetate). Other suitable hydrophobic polymers include polymers or copolymers derived from acrylic or methacrylic acid esters, copolymers of acrylic and methacrylic acid esters, zein, waxes, shellac and hydrogenated vegetable oils.

The hydroxypropylmethylcelluloses used according to the invention preferably have a viscosity (2 wt% solution at 20°C) of about 100 to 100,000 cps, preferably 100 to 30,000 cps. Especially suitable are Methocel K types or their equivalents. The hydroxypropylcelluloses used according to the invention preferably have a molecular weight of about 80,000 to 1,150,000, more preferably 80,000 to 600,000. Especially suitable is Klucel LF, which has a molecular weight of 100,000. The poly(ethylene oxide) used according to the invention preferably has a molecular weight of about 100,000 to 7,000,000, more preferably 900,000 to 7,000,000. Especially suitable is Polyox WSR Coagulant, which has a molecular weight of 5,000,000. The ethylcelluloses used according to the invention preferably have a viscosity of about 3 to 110 cps, more preferably 7 to 100 cps.

To ensure correct release kinetics, the controlled release preparation of this invention contains about 5 and 75% by weight, preferably about 20 and 50% by weight, more preferably about 30 to 45% by weight rate controlling polymer(s) and about 1 to 40% by weight, preferably about 3 to 25% by weight tiagabine.

The controlled release preparation according to the invention can preferably include auxiliary agents, such as diluents, lubricants and/or melting binders. Preferably, the excipients are selected to minimize the water content of the preparation. Preferably, the preparation includes an antioxidant.

Suitable diluents include pharmaceutically acceptable inert fillers such as microcrystalline cellulose, lactose, dibasic calcium phosphate, saccharides, and/or mixtures of any of the foregoing. The diluent is suitably a water soluble diluent. Examples of diluents include microcrystalline cellulose such as Avicel pH112, Avicel pH101 and Avicel pH102; lactose such as lactose monohydrate, lactose anhydrous, and Pharmatose DCL 21; dibasic calcium phosphate such as Emcompress; mannitol; starch; sorbitol; sucrose; and glucose. Diluents are carefully selected to match the specific formulation with attention paid to the compression properties. The diluent is preferably used in an amount of 10 to 90% by weight, preferably 25 to 65% by weight, of the controlled release preparation.

Suitable lubricants, including agents that act on the flowability of the powder to be compressed are, for example, colloidal silicon dioxide such as Aerosil 200; talc; stearic acid, magnesium stearate, and calcium stearate.

Suitable low temperature melting binders include polyethylene glycols such as PEG 6000; cetostearyl alcohol; cetyl alcohol; polyoxyethylene alkyl ethers; polyoxyethylene castor oil derivatives; polyoxyethylene sorbitan fatty acid esters; polyoxyethylene stearates; poloxamers; and waxes.

To improve the stability of the tiagabine in the controlled release preparation, an antioxidant compound can be included. Suitable antioxidants include sodium metabisulfite; tocopherols such as α, β, or δ -tocopherol, tocopherol esters and α-tocopherol acetate; ascorbic acid or a pharmaceutically acceptable salt thereof; ascorbyl palmitate; alkyl gallates such as propyl gallate, Tenox PG, Tenox s-1; sulphites or a pharmaceutically acceptable salt thereof; BHA; BHT; and monothioglycerol.

The controlled release preparation according to the invention preferably can be manufactured by blending tiagabine with the rate controlling polymer(s) and auxiliary excipients followed by direct compression. Other methods for manufacturing the preparation include melt granulation. Preferred melt granulation techniques include melt granulation of tiagabine together with the rate controlling polymer(s) and diluent(s) followed by compression of the granules and melt granulation of the tiagabine with subsequent blending of the tiagabine melt granules with the rate controlling polymer(s) and diluents followed by compression of the blend. As desired prior to compression, the blend and/or granulate can be screened and/or mixed with auxiliary agents until an easily flowable homogeneous mixture is obtained.

Oral dosage forms of the controlled release preparation according to the invention can be in the form of tablets or can be multiparticulate, such as in the form of pellets or mini-tablets. If desired, capsules such as hard or soft gelatin capsules, can contain the multiparticulates. If desired, the multiparticulate oral dosage forms can comprise a blend of at least two populations of pellets or mini-tablets having different controlled-release *in vitro* and/or *in vivo* tiagabine release profiles. If desired, one of the pellet or mini-tablet populations can comprise immediate release tiagabine multiparticulates, such as multiparticulates formed by conventional means.

If desired, the controlled release matrix tablets or multiparticulates of this invention can be coated with a controlled release polymer layer so as to provide additional controlled release properties. Suitable polymers that can be used to form this controlled release layer include the rate controlling polymers listed above.

As desired, the tablets, pellets or mini-tablets according to the invention can be provided with a light-protective and/or cosmetic film coating, for example, film-formers, pigments, anti-adhesive agents and plasticisers. Such a film former may consist of fast-dissolving constituents, such as low-viscosity hydroxypropylmethylcelluose, for example Methocel E5 or D14 or Pharmacoat 606 (Shin-Etsu). The film coating may also contain excipients customary in film-coating procedures, such as light-protective pigments, for example iron oxide, or titanium dioxide, anti-adhesive agents, for example talc, and also suitable plasticisers such as PEG 400, PEG 6000, diethyl phthalate or triethyl citrate.

Preferably, oral dosage forms according to the invention are suitable for twice-daily or, more preferably, once-daily administration of tiagabine. For example, once-daily oral dosage forms may contain from about 5 to 100 mg tiagabine, preferably 10 to 50 mg tiagabine, and most preferably 20 to 40 mg tiagabine to provide a therapeutic amount of tiagabine throughout the day in a controlled release fashion.

The rate controlling polymeric matrix of this invention may consist of a hydrogel matrix. For instance, tiagabine can be compressed into a dosage form containing a rate controlling polymer, such as HPMC, or mixture of polymers which when wet will swell to form a hydrogel. The rate of release of tiagabine from this dosage form is controlled both by diffusion from the swollen tablet mass and by erosion of the tablet surface over time. The rate of release of the tiagabine may be controlled both by the amount of polymer per tablet and by the inherent viscosities of the polymers used.

The tiagabine employed in the following examples is the monohydrate crystalline form for the hydrochloride salt of the R isomer. The raw material tiagabine hydrochloride monohydrate has high aqueous solubility and consists of fine needle shaped crystals which are relatively cohesive and do not provide good flow properties.

### Modes for Carrying Out the Invention

### Example 1: Direct Compression

Powder blends of tiagabine, the rate controlling polymer(s), and, if present, filler(s), diluent(s), lubricant(s) and/or antioxidation agent(s) are blended such as in a V-cone blender and tablet compression is carried out on a single station table press or a rotary tablet press, such as a Fette E1 rotary tablet press (Wilhelm Fette GMBH) for small scale production or a Horn (Horn of Noack Pharmatechnic GMBH) rotary table press or Fette P1000 for larger scale production. The dissolution rate for the resulting tablets is measured using USP Apparatus II (paddles) at 50 r.p.m. with 900 ml deionized water at 37°C as the dissolution media.

### Example 2: Direct Compression Formulations

Controlled release formulations having 30mg tiagabine in 300mg tablets and employing a variety of rate controlling polymers are prepared according to the small scale production procedures given in Example 1. Table 1 shows the formulation details and dissolution profiles for these formulations, which all exhibit controlled release over a 12 to 24-hour period. The release rates for the formulations containing different Methocel grades show rank order of K100LV (low viscosity) > K15M (intermediate viscosity) > K100M (high viscosity) while the release profile for the formulation containing Polyox, which has a relatively low moisture content compared to the Methocels, is almost equivalent to that of the K15M grade of Methocel.

**Table 1**

| Batch | 1 | 2 | 3 | 4 |
|---|---|---|---|---|
| Material | %wt | %wt | %wt | %wt |
| Tiagabine HCl | 11.50 | 11.50 | 11.50 | 11.50 |
| Monohydrate | | | | |
| Methocel K15M | 20.00 | - | - | - |
| Methocel K100M | - | - | 20.00 | - |
| Methocel K100LV | - | 20.00 | - | - |
| Polyox WSR | - | - | - | 20.00 |
| Klucel LF | 10.00 | 10.00 | 10.00 | 10.00 |
| Talc | 5.00 | 5.00 | 5.00 | 5.00 |
| Aerosil 200 | 0.20 | 0.20 | 0.20 | 0.20 |
| Sodium Metabisulphite | 0.25 | 0.25 | 0.25 | 0.25 |
| Avicel pH112 | 53.05 | 53.05 | 53.05 | 53.05 |

| Dissolution Profile (hrs) | mean % rel. | mean % rel. | mean % rel. | mean % rel. |
|---|---|---|---|---|
| 0.5 | 12.7 | 20.0 | 9.5 | 10.1 |
| 1 | 19.2 | 27.9 | 16.0 | 15.3 |
| 2 | 28.2 | 39.9 | 24.2 | 23.6 |
| 4 | 40.8 | 56.5 | 35.5 | 37.0 |
| 8 | 58.5 | 78.9 | 51.1 | 56.8 |
| 12 | 71.7 | 87.1 | 63.0 | 70.6 |
| 24 | 90.2 | 94.0 | 83.5 | 89.8 |

### Example 3: Direct Compression Formulations

Controlled release formulations having 10mg tiagabine in 300mg tablets and employing different types of diluents are prepared according to the small scale production procedures given in Example 1. Table 2 shows the formulations details and dissolution profiles for these formulations, which all exhibit controlled release over a 12 to 24-hour period.

**Table 2**

| Batch | 5 | 6 | 7 |
|---|---|---|---|
| Material | %wt | %wt | %wt |
| Tiagabine HCl Monohydrate | 3.82 | 3.82 | 3.82 |
| Methocel K15M | 10.00 | 10.00 | 10.00 |
| Methocel K100LV | 8.00 | 8.00 | 8.00 |
| Emcompress Anhydrous | - | - | 72.73 |
| Avicel pH 112 | - | 72.73 | - |
| Lactose | 72.73 | - | - |
| Talc | 5.00 | 5.00 | 5.00 |
| Aerosil 200 | 0.20 | 0.20 | 0.20 |
| Sodium Metabisulphite | 0.25 | 0.25 | 0.25 |

| Dissolution Profile (hrs) | mean % rel. | mean % rel. | mean % rel. |
|---|---|---|---|
| 0.5 | 18.9 | 29.1 | 15.8 |
| 1 | 27.6 | 38.4 | 23.2 |
| 2 | 40.9 | 51.5 | 33.6 |
| 4 | 58.9 | 64.5 | 47.4 |
| 8 | 82.2 | 79.4 | 66.5 |
| 12 | 89.8 | 87.7 | 78.8 |
| 24 | 90.6 | 92.6 | 92.2 |

### Example 4: Direct Compression Formulations

Controlled release formulations having 10mg tiagabine in 300mg tablets and employing different rate controlling polymers are prepared according to the larger scale production procedures given in Example 1. Table 3 shows the formulation details and dissolution profiles for these controlled release formulations.

### Example 5: Melt Granulation Type A - Diluent(s)/matrix polymer(s) added intragranularly

Tiagabine, the low temperature melting binder(s), the rate controlling polymer(s) and, if desired, filler(s), diluent(s) and/or antioxidation agent(s) are blended such as in a Gral 25L high speed granulator (GEI Collette) prior to the start of the melt process. The melt granulation is carried out with a thermal jacket and, if desired, under a nitrogen blanket. If desired, components such as the antioxidant a tocopherol can be added to the Gral bowl once the melt temperature has been reached. After the bowl temperature is lowered, lubricant(s) such as talc can be added. The resulting blend is tabletted and the dissolution rate for the tablets is measured using USP Apparatus II (paddles) at 50 r.p.m. with 900 ml deionized water at 37°C as the dissolution media.

**Table 3**

| Batch | 8 | 9 |
|---|---|---|
| Material | %wt | %wt |
| Tiagabine HCl.H₂O | 3.83 | 3.83 |
| Methocel K15M | 20 | - |
| Methocel K100LV | - | 25 |
| Klucel LF | 10 | 10 |
| Avicel pH112 | 60.53 | 55.53 |
| Aerosil 200 | 0.4 | 0.4 |
| Talc USP | 5.0 | 5.0 |
| Sodium Metabisulfite | 0.25 | 0.25 |
| Theoretical potency | 10 | 10 |
| (mg/tab) | | |
| Punch size/shape | 14x7 mm scored oval | 14x7 mm scored oval |
| Mean Tablet Hardness (N) | 106 | 118 |

| Dissolution Profile (hrs) | mean % release | mean % release |
|---|---|---|
| 0.5 | 18.4 | 19.2 |
| 1 | 28.0 | 26.3 |
| 2 | 39.8 | 36.8 |
| 4 | 54.2 | 55.5 |
| 6 | 63.2 | 69.3 |
| 8 | 69.6 | 76.6 |
| 10 | 74.7 | 81.5 |
| 16 | 85.0 | |
| 22 | 89.0 | 91.3 |

### Example 6: Melt Granulation Formulations- Type A

Controlled release formulations having 10mg. tiagabine in 300mg tablets containing 20%wt and 30%wt Methocel K15M are prepared according to the procedures given in Example 5. Table 4 shows the formulation details and dissolution profiles for these controlled release formulations.

**Table 4**

| Batch | 10 | 11 | 12 | 13 |
|---|---|---|---|---|
| Material | %wt | | %wt | |
| Tiagabine HCl.H₂O | 3.83 | | 3.83 | |
| PEG 6000 | 6.25 | | 6.25 | |
| Methocel K15M | 20.00 | | 30.00 | |
| Klucel LF | 10.00 | | 15.00 | |
| Pharmatose DCL 21 | 10.78 | | 10.78 | |
| Lactose Monohydrate | | | | |
| Avicel pH112 | 43.81 | | 28.81 | |
| α-Tocopherol | 0.33 | | 0.33 | |
| Talc USP | 5.00 | | 5.00 | |
| Theoretical potency (mg/tab) | 10 | 10 | 10 | 10 |
| Punch size/shape | 13 x 8 mm oval | 14 x7 mm caplet | 13 x 8 mm oval | 14 x 7 mm caplet |
| Mean Tablet Hardness (N) | 50.1 | 40.3 | 47.3 | 40.3 |

| Dissolution Profile (hrs) | Mean % Rel. | Mean % Rel. | Mean % Rel. | Mean % Rel. |
|---|---|---|---|---|
| 0.5 | 9.1 | 10.5 | 8.3 | 8.3 |
| 1 | 13.8 | 15.5 | 12.6 | 13.1 |
| 2 | 20.9 | 23.4 | 19.1 | 20.3 |
| 4 | 31.3 | 35.7 | 28.9 | 31.6 |
| 8 | 49.0 | 57.2 | 45.0 | 51.1 |
| 12 | 63.9 | 73.9 | 59.6 | 67.2 |
| 24 | 91.6 | 98.4 | 89.3 | 98.7 |

### Example 7: Melt Granulation Type B - Diluent(s)/matrix polymers(s) added extragranularly

Tiagabine and other pharmaceutical excipients such as low temperature melting binders and optionally an antioxidant(s) are blended such as in a 24L Gral high speed granulator prior to the start of the melt process. The melt granulation is carried out with a thermal jacket and, if desired, under a nitrogen blanket. If desired, components such as the antioxidant a-tocopherol are added to the Gral bowl once the melt temperature has been reached. Following melt granulation, the cooled granulate can optionally be screened such as by passing it through a sieve. The granulate is blended with the diluent(s), rate controlling polymers(s) and, optionally other excipients such as silicon dioxide and talc. The resulting blend is tabletted and the dissolution rate for the tablets is measured using USP Apparatus II (paddles) at 50 r.p.m. with 900 ml deionized water at 37°C as the dissolution media.

### Example 8: Melt Granulation Formulations- Type B

Controlled release formulations having 10mg tiagabine in 300mg tablets containing Methocel K15M or Polyox WSR are prepared according to the procedures given in Example 7 and tabletted using a Fette E1 single station tablet press. The tiagabine granulate for Batches 14-17 was screened through a 600mm sieve prior to tableting. Table 5 shows the formulation details and dissolution profiles for these controlled release formulations.

**Table 5**

| Batch | 14 | 15 | 16 | 17 | 18 | 19 | 20 |
|---|---|---|---|---|---|---|---|
| Tiagabine | %wt | | | | | | |
| Granulate | | | | | | | |
| Tiagabine | 23.95 | | | | | | |
| HCl.H₂0 | | | | | | | |
| PEG 6000 | 6.25 | | | | | | |
| α-Tocopherol | 2.395 | | | | | | |
| Anhydrous Lactose | 67.405 | | | | | | |

| Materials | %wt | %wt | %wt | %wt | %wt | %wt | %wt |
|---|---|---|---|---|---|---|---|
| Tiagabine | 15.987 | 15.987 | 15.987 | 15.98 | 15.987 | 15.987 | 15.987 |
| granulate | | | | | | | |
| Methocel K15M | 20.00 | 20.00 | 30.00 | 30.00 | 20.00 | 30.00 | - |
| Polyox WSR | - | - | - | - | - | - | 20.00 |
| Klucel LF | 10.00 | 10.00 | 15.00 | 15.00 | 10.00 | 15,00 | 10.00 |
| Talc | 5.00 | 5.00 | 5.00 | 5.00 | 5.00 | 5.00 | 5.00 |
| Aerosil 200 | 0.40 | 0.40 | 0.40 | 0.40 | - | - | - |
| Avicel pH112 | 48.613 | 48.613 | 33.613 | 33.613 | 49.013 | 34.01 | 49.013 |
| Theoretical potency | 10 | 10 | 10 | 10 | 10 | 10 | 10 |
| Punch size/shape | 13x8 mm oval | 14x7 mm caplet | 13x8 mm oval | 14x7 mm caplet | 13x8 mm oval | 13x8 mm oval | 13x8 mm oval |
| Mean Tab. Hardness | 93.5 | 93.0 | 102.2 | 89.6 | 101.5 | 102.1 | |

| Dissolution Profile (hrs) | Mean % Rel. | Mean % Rel. | Mean % Rel. | Mean % Rel. | Mean % Rel. | Mean % Rel. | Mean % Rel. |
|---|---|---|---|---|---|---|---|
| 0.5 | 11.6 | 11.9 | 7.8 | 7.6 | 19.3 | 7.8 | 11.4 |
| 1 | 17.0 | 15.1 | 12.0 | 11.5 | 26.2 | 11.5 | 16.5 |
| 2 | 26.3 | 22.9 | 17.9 | 17.5 | 31.6 | 17.8 | 26.1 |
| 4 | 40.6 | 35.6 | 27.1 | 27.2 | 56.3 | 31.6 | 42.9 |
| 8 | 59.7 | 53.8 | 41.2 | 43.0 | 73.0 | 50.0 | 61.5 |
| 12 | 73.1 | 67.0 | 52.2 | 55.3 | 83.3 | 66.6 | 73.2 |
| 24 | 92.2 | 86.9 | 72.6 | 76.4 | 93.6 | 94.7 | 85.8 |

### Example 9: Melt Granulation Formulations- Type B

Controlled release formulations having 10mg tiagabine in 300mg tablets containing Methocel K15M or Polyox WSR are prepared according to the procedures given in Example 7. Batches 21 and 22 were screened through a 600mm sieve and tabletted using a Horn rotary tablet press. Table 6 shows the formulation details and dissolution profiles for these controlled release formulations.

**Table 6**

| Batch | 21 | 22 |
|---|---|---|
| Tiagabine Granulate | %wt | |
| Tiagabine HCl.H₂O | 23.88 | |
| PEG 6000 | 6.25 | |
| α-Tocopherol Acetate | 2.388 | |
| Pharmatose DC L21 | 67.482 | |

| Materials | %wt | %wt |
|---|---|---|
| Tiagabine granulate | 16 | 16 |
| Methocel K 15M | 30 | 20 |
| Klucel LF | 15 | 10 |
| Talc | 5.0 | 5.0 |
| Aerosil 200 | 0.4 | 0.4 |
| Avicel pH112 | 33.6 | 48.6 |
| Theoretical potency (mg/tab) | 10 | 10 |
| Punch size/shape | 14x7 mm scored oval | 14x7 mm scored oval |
| Mean Tab. Hardness | 94 | 99 |

| Dissolution Profile (hrs) | mean % release | mean % release |
|---|---|---|
| 0.5 | 8.1 | 11.7 |
| 1 | 13.2 | 16.3 |
| 2 | 21.8 | 28.8 |
| 4 | 35.0 | 44.8 |
| 6 | 46.5 | 57.8 |
| 8 | 57.4 | 68.3 |
| 10 | 66.4 | 85.5 |
| 16 | 88.1 | |
| 22.0 | 99.0 | 103.1 |

### Example 10: Bioavailability Study

An open label, randomized, four period cross-over study was undertaken in 12 healthy male Caucasian volunteers aged 18 to 45 (11 subjects completed) evaluating the bioavailability of three controlled release 10mg tiagabine tablet formulations A, B and C made according to this invention compared to a 10mg conventional instant release tablet formulation D (Gabitril (Gabitril is a trade mark); Novo Nordisk). Dosing occurred after an overnight fast and four hours prior to a meal. Blood samples were collected up to 36 hours post dosing with a one week washout period separating the doses. All blood samples were collected in heparinized tubes, stored immediately at 4°C and centrifuged within an hour. After separation, the plasma was stored at - 18°C or below pending assay. Plasma samples were assayed using a validated HPLC-assay.

Figure 1 shows the plasma concentration of tiagabine following administration of formulation A (Batch 8 of Example 4), formulation B (Batch 21 of Example 9), formulation C (Batch 22 of Example 9) and formulation D (Gabitril (Gabitril is a trade mark)). Non-compartmental pharmacokinetic analysis of the plasma concentration data resulted in the parameters presented in Table 7. The time during which the plasma concentration was greater than 50% of the maximum plasma concentration (mean T₅₀) for formulations A, B and C was calculated to be 22.7 hours, 24.7 hours and 21.1 hours, respectively. Formulations A, B and C provide a good extension of plasma drug levels with little loss in relative bioavailability compared to the reference immediate release product. Additionally, these controlled release products effect minimal peak to trough fluctuations in tiagabine blood plasma levels.

**Table 7**

| Formulation | N | Cₘₐₓ (ng/ml) | tₘₐₓ (h) | AUC₀₋₃₆ (ng□h/ml) | t_{1/2} (h) |
|---|---|---|---|---|---|
| A | 11 | 52.5±12.2 | 8.27±3.72 | 1203±297 | 10.5 |
| B | 11 | 43.7±10.3 | 12.91±4.55 | 1255±325 | 11.7 |
| C | 11 | 51.2±1.2 | 7.91±5.38 | 1136±360 | 8.9 |
| D | 11 | 191±49.4 | 0.77±0.31 | 1337±272 | 9.3 |

Projected steady state tiagabine levels were determined based upon the mean single dose data for controlled release formulations A, B, C and D (10mg dosing). Table 8 shows the steady state pharmacokinetic parameters based upon these projections for formulations A, B and C. Figure 2 illustrates the projected steady state plasma tiagabine profiles for formulations A, B and C at 30mg dosing once daily compared to the conventional formulation D dosed 10mg t.i.d.

**Table 8**

| Parameter | Formulation A | Formulation B | Formulation C |
|---|---|---|---|
| AUC_{ss (0-24)} | 1248.7 | 1125.1 | 1203.1 |
| Cₐᵥ | 52.0 | 46.9 | 50.1 |
| Cₘₐₓ | 71.4 | 60.5 | 70.9 |
| Cmin | 32.1 | 36.8 | 34.3 |
| (Cₘₐₓ-Cₘᵢₙ)/Cₐᵥ | 0.76 | 0.51 | 0.73 |

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

## Claims

1. A controlled release oral pharmaceutical preparation comprising a therapeutically effective amount of tiagabine or a pharmaceutically acceptable salt thereof dispersed in a rate controlling polymeric matrix comprising at least one rate controlling polymer, which preparation provides therapeutically effective plasma levels of tiagabine for a period of at least 12 hours, and which provides a mean *in vitro* dissolution profile in aqueous media such that about 5 to 40% of the tiagabine is released after 1 hour; about 25 to 65% of the tiagabine is released after 4 hours; about 55 to 95% of the tiagabine is released after 10 hours and about 80 to 100% of the tiagabine is released after 22 hours, when carried out in a USP Apparatus (II) (paddles) at 50 r.p.m. in deionised water at 37°C as dissolution medium.

2. A preparation according to Claim 1, wherein the preparation releases tiagabine *in vivo* such that the duration over which the tiagabine plasma concentration is equal to or greater than 50% of the peak plasma concentration is 10 hours or greater.

3. A preparation according to Claim 1 or 2, wherein the *in vivo* maximum plasma concentration minus the minimum plasma concentration divided by the average plasma concentration taken over the effective period is less than 0.80.

4. A preparation according to any preceding claim, which provides therapeutic levels of tiagabine over a 24 hour period for once-daily administration.

5. A preparation according to any preceding claim, wherein the duration over which the tiagabine plasma concentration is equal to or greater than 50% of the peak concentration is at least 15 hours.

6. A preparation according to any preceding claim, wherein the duration over which the tiagabine plasma concentration is equal to or greater than 50% of the peak concentration is at least 20 hours.

7. A preparation according to any one of Claims 3 - 6, wherein the *in vivo* maximum plasma concentration minus the minimum plasma concentration divided by the average plasma concentration taken over the effective period is less than 0.60.

8. A preparation according to any preceding claim, wherein the preparation provides a mean dissolution profile in aqueous media such that about 10 to 30% of the tiagabine is released after 1 hour; about 30 to 60% of the tiagabine is released after 4 hours; about 60 to 90% of the tiagabine is released after 10 hours and about 85 to 100% of the tiagabine is released after 22 hours.

9. A preparation according to any preceding claim, wherein the at least one rate controlling polymer is selected from hydroxypropylmethylcellulose, hydroxyalkylcellulose, alkylcellulose, poly(ethylene)oxide, carboxymethylcellulose, hydrophilic cellulose derivatives, polyethylene glycols, polyvinylpyrrolidone, or mixtures thereof.

10. A preparation according to any preceding claim, wherein the at least one rate controlling polymer is selected from hydroxypropylmethylcellulose having a viscosity of about 100 to 100,000 cps, hydroxypropylcellulose having a molecular weight of about 80,000 to 1,150,000, ethylcellulose having a viscosity of about 3 to 110 cps and poly(ethylene)oxide having a molecular weight of about 100,000 to 7,000,000 or mixtures thereof.

11. A preparation according to any preceding claim, wherein the at least one rate controlling polymer constitutes from about 5 to 75% by weight of the preparation.

12. A preparation according to any preceding claim, wherein the at least one rate controlling polymer constitutes from about 20 to 50% by weight of the preparation.

13. A preparation according to any preceding claim, wherein the at least one rate controlling polymer constitutes from about 30 to 45% by weight of the preparation.

14. A preparation according to any preceding claim, wherein the therapeutically effective amount of tiagabine or a pharmaceutically acceptable salt thereof is from about 5 to 100 mg.

15. A preparation according to any preceding claim, further comprising a diluent constituting from 10 to 90 % by weight of the preparation.

16. A preparation according to any preceding claim, wherein the rate controlling polymeric matrix is a hydrogel.

17. A preparation according to any preceding claim, wherein the at least one rate controlling polymer comprises from 19 to 31 % by weight of a hydroxypropylmethylcellulose and from 9 to 15 % by weight of a hydroxypropylcellulose.

18. An oral dosage form suitable for once or twice daily administration containing a controlled release oral pharmaceutical preparation according to any one of Claims 1 to 17.

19. An oral dosage form according to Claim 18, which is in the form of tablets.

20. An oral dosage form according to Claim 18, which is in the form of pellets or mini-tablets.

21. An oral dosage form according to Claim 18, which is in the form of a blend of at least two populations of pellets or mini-tablets, wherein each population has a different controlled-release dissolution profile.

22. An oral dosage form according to any one of Claims 18 to 21, further comprising a controlled release polymer layer coated on the preparation.

23. An oral dosage form according to any one of Claims 18 to 22, further comprising a light-protective or cosmetic film coated on the preparation.

24. An oral dosage form according to Claim 20 or Claim 21, further comprising immediate release pellets or mini-tablets containing tiagabine or a pharmaceutically acceptable salt thereof.

## Patentansprüche

1. Orale pharmazeutische Präparation mit kontrollierter Wirkstoffreisetzung, enthaltend eine therapeutisch wirksame Menge von Tiagabin oder von einem pharmazeutisch verträglichen Salz hiervon, dispergiert in einer geschwindigkeitskontrollierenden Polymermatrix, die mindestens ein geschwindigkeitskontrollierendes Polymeres enthält, wobei die Präparation therapeutisch wirksame Plasmaspiegel von Tiagabin für einen Zeitraum von mindestens 12 h bereitstellt und in einem wäßrigen Medium ein mittleres *in-vitro*-Auflösungsprofil bereitstellt, derart, daß etwa 5 bis 40 % des Tiagabins nach 1 h freigesetzt sind; etwa 25 bis 65 % des Tiagabins nach 4 h freigesetzt sind; etwa 55 bis 95 % des Tiagabins nach 10 h freigesetzt sind und etwa 80 bis 100 % des Tiagabins nach 22 h freigesetzt sind, bei Durchführung in einer USP-Vorrichtung (II) (Paddel) bei 50 U/min in deionisiertem Wasser bei 37° C als Auflösungsmedium.

2. Präparation nach Anspruch 1, wobei die Präparation Tiagabin *in vivo* derart freisetzt, daß die Dauer, während der die Tiagabin-Plasmakonzentration gleich oder größer ist als 50 % der Plasma-Peakkonzentration, 10 h oder mehr beträgt.

3. Präparation nach Anspruch 1 oder 2, wobei die maximale Plasmakonzentration minus der minimalen Plasmakonzentration dividiert durch die mittlere Plasmakonzentration während der Wirkungsdauer *in vivo* kleiner ist als 0,80.

4. Präparation nach einem vorhergehenden Anspruch, die über einen 24-h-Zeitraum therapeutische Konzentrationen von Tiagabin zur einmal täglichen Verabreichung bereitstellt.

5. Präparation nach einem vorhergehenden Anspruch, wobei die Dauer, während der die Tiagabin-Plasmakonzentration gleich oder größer ist als 50 % der Peakkonzentration, mindestens 15 h beträgt.

6. Präparation nach einem vorhergehenden Anspruch, wobei die Dauer, während der die Tiagabin-Plasmakonzentration gleich oder größer ist als 50 % der Peakkonzentration, mindestens 20 h beträgt.

7. Präparation nach einem der Ansprüche 3 - 6, wobei die maximale Plasmakonzentration minus der minimalen Plasmakonzentration dividiert durch die mittlere Plasmakonzentration während der Wirkungsdauer *in vivo* kleiner ist als 0,60.

8. Präparation nach einem vorhergehenden Anspruch, wobei die Präparation in einem wäßrigen Medium ein mittleres Auflösungsprofil bereitstellt, derart, daß etwa 10 bis 30 % des Tiagabins nach 1 h; etwa 30 bis 60 % des Tiagabins nach 4 h; etwa 60 bis 90 % des Tiagabins nach 10 h und etwa 85 bis 100 % des Tiagabins nach 22 h freigesetzt sind.

9. Präparation nach einem vorhergehenden Anspruch, wobei das mindestens eine geschwindigkeitskontrollierende Polymere ausgewählt ist aus Hydroxypropylmethylcellulose, Hydroxyalkylcellulose, Alkylcellulose, Poly(ethylen)oxid, Carboxymethylcellulose, hydrophilen Cellulose-Derivaten, Polyethylenglycolen, Polyvinylpyrrolidon oder Gemischen hiervon.

10. Präparation nach einem vorhergehenden Anspruch, wobei das mindestens eine geschwindigkeitskontrollierende Polymere ausgewählt ist aus Hydroxypropylmethylcellulose mit einer Viskosität von etwa 100 bis etwa 100000 cps, Hydroxypropylcellulose mit einem Molekulargewicht von etwa 80000 bis 1150000, Ethylcellulose mit einer Viskosität von etwa 3 bis 110 cps und Poly(ethylen)oxid mit einem Molekulargewicht von etwa 100000 bis 7000000 oder Gemischen hiervon.

11. Präparation nach einem vorhergehenden Anspruch, wobei das mindestens eine geschwindigkeitskontrollierende Polymere etwa 5 bis 75 Gew.-% der Präparation ausmacht.

12. Präparation nach einem vorhergehenden Anspruch, wobei das mindestens eine geschwindigkeitskontrollierende Polymere etwa 20 bis 50 Gew.-% der Präparation ausmacht.

13. Präparation nach einem vorhergehenden Anspruch, wobei das mindestens eine geschwindigkeitskontrollierende Polymere etwa 30 bis 45 Gew.-% der Präparation ausmacht.

14. Präparation nach einem vorhergehenden Anspruch, wobei die therapeutisch wirksame Menge von Tiagabin oder einem pharmazeutisch verträglichen Salz hiervon etwa 5 bis 100 mg beträgt.

15. Präparation nach einem vorhergehenden Anspruch, die außerdem ein Verdünnungsmittel enthält, das 10 bis 90 Gew.-% der Präparation ausmacht.

16. Präparation nach einem vorhergehenden Anspruch, wobei die geschwindigkeitskontrollierende Polymermatrix ein Hydrogel ist.

17. Präparation nach einem vorhergehenden Anspruch, wobei das mindestens eine geschwindigkeitskontrollierende Polymere 19 bis 31 Gew.-% einer Hydroxypropylmethylcellulose und 9 bis 15 Gew.-% einer Hydroxypropylcellulose enthält.

18. Orale Dosierungsform, die zur ein- oder zweimaligen täglichen Verabreichung geeignet ist, enthaltend eine kontrolliert freisetzende orale pharmazeutische Präparation nach einem der Ansprüche 1 bis 17.

19. Orale Dosierungsform nach Anspruch 18, die in Form von Tabletten vorliegt.

20. Orale Dosierungsform nach Anspruch 18, die in Form von Pellets oder Minitabletten vorliegt.

21. Orale Dosierungsform nach Anspruch 18, die in Form eines Gemisches von mindestens zwei Populationen von Pellets oder Minitabletten vorliegt, wobei jede Population ein unterschiedliches kontrolliert freisetzendes Auflösungsprofil aufweist.

22. Orale Dosierungsform nach einem der Ansprüche 18 bis 21, die außerdem eine kontrolliert freisetzende Polymerschicht aufweist, mit der die Präparation überzogen ist.

23. Orale Dosierungsform nach einem der Ansprüche 18 bis 22, die außerdem einen Lichtschutz- oder kosmetischen Film aufweist, mit dem die Präparation überzogen ist.

24. Orale Dosierungsform nach Anspruch 20 oder 21, die außerdem sofort freisetzende Pellets oder Minitabletten enthält, die Tiagabin oder ein pharmazeutisch verträgliches Salz hiervon enthalten.

## Revendications

1. Préparation pharmaceutique retard administrable par voie orale, comprenant une quantité thérapeutiquement efficace de tiabagine ou d'un sel pharmaceutiquement acceptable de celle-ci dispersé dans une matrice polymère réglant la vitesse qui comprend au moins un polymère réglant la vitesse, préparation qui assure des taux plasmatiques thérapeutiquement efficaces de tiabagine pendant une durée d'au moins 12 heures et qui assure un profil de dissolution moyen en milieu aqueux *in vitro* tel, qu'environ 5 à 40 % de tiabagine soient libérés au bout d'une heure ; environ 25 à 65 % de tiabagine soient libérés au bout de 4 heures ; environ 55 à 95 % de tiabagine soient libérés au bout de 10 heures ; et environ 80 à 100 % de tiabagine soient libérés au bout de 22 heures, lorsque l'essai est pratiqué dans un appareil USP (II) (à palettes) à 50 t/min dans de l'eau désionisée à 37°C en tant que milieu de dissolution.

2. Préparation selon la revendication 1, dans laquelle la préparation libère la tiabagine *in vivo* à une allure telle, que la durée pendant laquelle le taux plasmatique de tiabagine est supérieur ou égal à 50 % du taux plasmatique pic soit de 10 heures ou plus.

3. Préparation selon la revendication 1 ou 2, dans laquelle le taux plasmatique maximum *in vivo* moins le taux plasmatique minimum *in vivo* divisé par le taux plasmatique moyen déterminé pendant la période efficace est inférieur à 0,80.

4. Préparation selon l'une quelconque des revendications précédentes, qui assure des taux thérapeutiques de tiabagine pendant une durée de 24 heures pour une administration une fois par jour.

5. Préparation selon l'une quelconque des revendications précédentes, dans laquelle la durée pendant laquelle le taux plasmatique de tiabagine est supérieur ou égal à 50 % du taux plasmatique pic est d'au moins 15 heures.

6. Préparation selon l'une quelconque des revendications précédentes. dans laquelle la durée pendant laquelle le taux plasmatique de tiabagine est supérieur ou égal à 50 % du taux plasmatique pic est d'au moins 20 heures.

7. Préparation selon l'une quelconque des revendications 3 à 6, dans laquelle le taux plasmatique maximum *in vivo* moins le taux plasmatique minimtun *in vivo* divisé par le taux plasmatique moyen déterminé pendant la période efficace est inférieur à 0,60.

8. Préparation selon l'une quelconque des revendications précédentes, dans laquelle la préparation assure un profil de dissolution moyen en milieu aqueux tel, qu'environ 10 à 30 % de tiabagine soient libérés au bout d'une heure : environ 30 à 60 % de tiabagine soient libérés au bout de 4 heures ; environ 60 à 90 % de tiabagine soient libérés au bout de 10 heures : et environ 85 à 100 % de tiabagine soient libérés au bout de 22 heures.

9. Préparation selon l'une quelconque des revendications précédentes. dans laquelle le «au moins un» polymère réglant la vitesse est choisi parmi les hydroxypropylméthylcelluloses, les hydroxyalkylcelluloses, les alkylcelluloses, les poly(oxyde d'éthylène), la carboxyméthylcellulose. les dérivés cellulosiques hydrophiles, les polyéthylèneglycols, la polyvinylpyrrolidone. ou des mélanges de ces composés.

10. Préparation selon l'une quelconque des revendications précédentes. dans laquelle le «au moins un» polymère réglant la vitesse est choisi parmi une hydroxypropylméthylcellulose ayant une viscosité d'environ 100 à 100 000 cps, une hydroxypropylcellulose ayant une masse moléculaire d'environ 80 000 à 1 150 000, une éthylcellulose ayant une viscosité d'environ 3 à 110 cps et un poly(oxyde d'éthylène) ayant une masse moléculaire d'environ 100 000 à 7 000 000, ou des mélanges de ces composés.

11. Préparation selon l'une quelconque des revendications précédentes, dans laquelle le «au moins un» polymère réglant la vitesse constitue environ 5 à 75% en poids de la préparation.

12. Préparation selon l'une quelconque des revendications précédentes, dans laquelle le «au moins un» polymère réglant la vitesse constitue environ 20 à 50% en poids de de la préparation.

13. Préparation selon l'une quelconque des revendications précédentes, dans laquelle le «au moins un» polymère réglant la vitesse constitue environ 30 à 45% en poids de de la préparation.

14. Préparation selon l'une quelconque des revendications précédentes. dans laquelle la quantité thérapeutiquement efficace de tiabagine ou d'un sel pharmaceutiquement acceptable de celle-ci est d'environ 5 à 100 mg.

15. Préparation selon l'une quelconque des revendications précédentes. qui comprend en outre un diluant constituant environ 10 à 90% en poids de la préparation.

16. Préparation selon l'une quelconque des revendications précédentes, dans laquelle la matrice de polymère réglant la vitesse est un hydrogel.

17. Préparation selon lune quelconque des revendications précédentes. dans laquelle le «au moins un» polymère réglant la vitesse comprend de 19 à 31% en poids d'une hydroxypropylméthylcellulose et de 9 à 15 % en poids d'une hydroxypropylcellulose.

18. Forme galénique destinée à la voie orale, pour une administration une ou deux fois par jour, contenant une préparation pharmaceutique retard destinée à la voie orale selon l'une quelconque des revendications 1 à 17.

19. Forme galénique destinée à la voie orale selon la revendication 18, qui se présente sous forme de comprimés.

20. Forme galénique destinée à la voie orale selon la revendication 18, qui se présente sous forme de granules ou de mini-comprimés.

21. Forme galénique destinée à la voie orale selon la revendication 18, qui se présente sous forme d'un mélange d'au moins deux groupes de granules ou de mini-comprimés, chaque groupe ayant un profil de dissolution à libération progressive différent.

22. Forme galénique destinée à la voie orale selon l'une quelconque des revendications 18 à 21, qui comprend en outre une couche de polymère à libération contrôlée appliquée sur la préparation.

23. Forme galénique destinée à la voie orale selon l'une quelconque des revendications 18 à 22, qui comprend en outre un film protégeant de la lumière ou un film cosmétique sur la préparation.

24. Forme galénique destinée à la voie orale selon la revendication 20 ou 21. comprenant en outre des granules ou mini-comprimés à libération immédiate contenant de la tiabagine ou un sel pharmaceutiquement acceptable de celle-ci.
